Europäisches Patentamt

**European Patent Office**

**Office européen des brevets**

(11) Publication number: **0 304 177**
**A2**

## EUROPEAN PATENT APPLICATION

(21) Application number: 88306869.4

(22) Date of filing: 26.07.88

(51) Int. Cl.⁴: **C12M 1/00 , C12N 1/00**

(30) Priority: 28.07.87 GB 8717831

(43) Date of publication of application:
**22.02.89 Bulletin 89/08**

(84) Designated Contracting States:
**DE FR GB IT**

(71) Applicant: **BIOTECHNA LIMITED**
**81 Wimpole Street**
**London WIM 7DB(GB)**

(72) Inventor: **Robinson, Lee Fisher**
**3, Athenaeum Hall Vale of Health**
**London NW3 1AP(GB)**
Inventor: **Morrison, Angus William**
**East Stansted Tilburstow Hill**
**South Godstone Surrey RN9 8NA(GB)**

(74) Representative: **BATCHELLOR, KIRK & EYLES**
**2 Pear Tree Court Farringdon Road**
**London EC1R 0DS(GB)**

(54) Improvements relating to biomass production.

(57) In a method of biomass culture production under sterile conditions in a closed system, a quantity of the synthesis fluid is withdrawn from the system, solid biomass product is filtered from the fluid, nutrient make-up liquor is added to the filtrate and, after sterilising the filtrate, it is returned to the closed system.

EP 0 304 177 A2

## IMPROVEMENTS RELATING TO BIOMASS PRODUCTION

This invention relates to biomass production. Biosynthesis routes involving biomass culture for obtaining commercially valuable products have been in use for a considerable time. However, many of these routes suffer from problems, particularly the production of species of hostile bacteria. The sterile conditions which should exist in the system operating the biomass culture are often lost in the process of removing the commercially valuable product from the system and in introducing make-up liquor into the system.

An object of the present invention is to provide a biomass production process in which this difficulty is overcome.

According to the present invention, in a method of operating biomass culture under sterile conditions in a closed system, a quantity of the synthesis fluid is withdrawn from the closed system; solid biomass product is filtered from the fluid; nutrient make-up liquor is added to the filtrate and, after being sterilised, the filtrate is returned to the closed system.

In this way, the withdrawal of the biomass product from the fluid and the addition of nutrient liquor to the filtrate takes place outside the closed system and the filtrate is sterilised before it is returned to the closed system. Thus, the sterile conditions in the closed system are not lost.

After the filtrate has been sterilised and cooled, carbon dioxide may be introduced into it prior to it being returned to the closed system.

The closed system in which the biomass production takes place may be a bioreactor comprising an upstanding core structure with a substantially transparent tube wound on the core structure so that, in use, the biomass synthesis mixture flows continuously through the tube, the exterior of which is exposed to light.

In order that the invention may be more readily understood, it will now be described, by way of example only, with reference to the accompanying drawings, in which:-

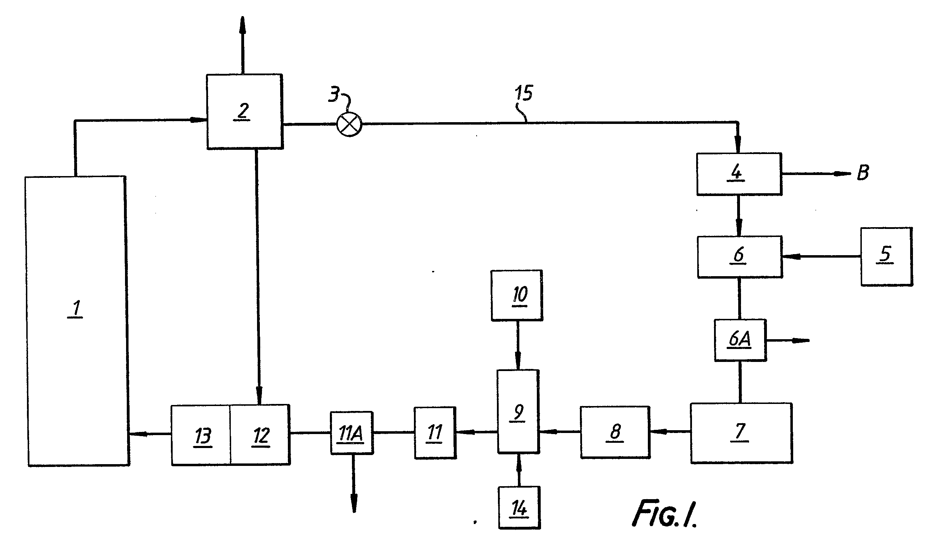

Figure 1 is a block diagram of a biomass production system; and

Figure 2 is a diagrammatic view of a photobioreactor.

Reference numeral 1 indicates a container in which a biomass culture takes place under sterile conditions at a processing temperature of, say, 28 - 30° C. The synthesis fluid is continuously pumped from the container to a disengagement column 2 and back to the container 1 by way of a recycle pump 12 with a heat exchanger 13 if necessary. In the disengagement column 2, gas generated during the process is allowed to escape to atmosphere.

A valve 3 is located in a pipe 15 leading from the disengagement column and this valve is opened to allow a bleed-off of the fluid from the disengagement column. This fluid may be a constant bleed of, say, 5 - 10% of the circulating fluid in the closed system or the valve may operate at set intervals, say, hourly, to deliver 10 - 20% of the total fluid from the container 1. The fluid flowing along the pipe 15 enters a filtration system 4 from which the solid biomass product B, such as spirulina, is removed from the liquid filtrate. The filtration system can be one of any of a number of known types, i.e. rotary vacuum, microstrainer, edge filters or simple Buchner types or even filter presses. The particular form of filtration system is chosen for the particular type of biomass culture which is being produced. From the filtration system the filtrate is passed to a mixing tank 6 into which additional nutrients are added from a source 5. This nutrient is in the form of a liquor and is added to the filtrate in the tank according to an automatic analyser which indicates the levels of carbonate, phosphate, nitrate or other chemicals as appropriate in the filtrate.

As the sterile conditions in the container 1 may not be retained in the filtration system 4 and the mixing tank 6, the filtrate with the added nutrients are passed through a steriliser 7 which serves to kill off all hostile bacteria and other living substances contained within the filtrate. Prior to entering the steriliser, the filtrate is conveniently passed through an ultra filtration unit in the form of a micro-filter 6A to remove any remaining solids of at least 0.2 microns. The sterilisation system is usually a steam steriliser. Any residual biomass materials and killed bacteria and other materials may be filtered off from the sterilised liquid. From the steriliser the liquid is passed through a cooler 8 to reduce the temperature to approximately 20° C. If desired, the cooled liquid may then be introduced to an absorption system 9 where carbon dioxide form a source 10 is added in the appropriate quantity to react with the alkaline hydroxide present in the liquid to raise the carbonate and bicarbonate in the solution to the required level. The amount of carbon dioxide which is added can be automatically controlled by an analyser operating through an electrically controlled relay system. This absorption unit 9 can be pressurised if desired to ensure $CO_2$ solution. Sterile innoculum may be introduced into the absorption unit 9 from a source 14. The treated liquor is then pumped by a dosing pump 11 into the inlet side of the recycle pump 12 and it is returned into the closed system of the container 1 and the disengagement column 2. A further ultra filtration unit in the form of a micro-filter 11A to remove any solids of at least 0.2 microns may be positioned between the dosing pump and the recycle pump. A heat exchanger 13 may be positioned between the recycle pump 12 and the container 1 to ensure that the processing temperature is at the required level.

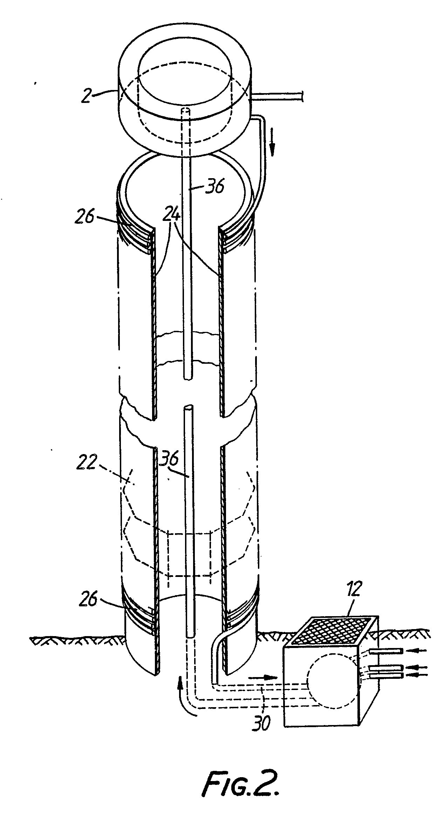

The closed system including the container 1 may take the form of the bioreactor shown in Figure 2.

Referring to Figure 2, an upstanding core structure 22, shown dotted, is provided with a substantially continuous outer surface and, on this surface, there is a layer 24 of a light reflective material, such as aluminium foil. A spirally wound tube 26 of substantially transparent material, such as polyethylene, is supported on the layer 24 by pegs or the like.

A pump 12 receives the synthesis fluid from one end 30 of the tube 26 and pumps it back up a tube 36 extending upwardly in the core structure to a disengagement column 2. The column contains a purge system to remove gas generated during the process. The fluid flows from the header into the spirally wound tube 26.

The advantage of the present invention is that the main photosynthetic closed system remains in sterile conditions and the production of wanted bioimass is produced continuously in the sterile conditions. The biomass is removed by a bypass unit where the biomass is removed from the liquid and additional nutrient liquor is added to the liquid. The liquor is sterilised before it is returned to the closed system.

## Claims

1. A method of operating biomass culture under sterile conditions in a closed system, characterised in that a quantity of the synthesis fluid is withdrawn from the closed system; solid biomass product is filtered from the fluid; nutrient make-up liquor is added to the filtrate and, after being sterilised, the filtrate is returned to the closed system.

2. A method as claimed in claim 1, characterised in that a proportion of the fluid in the closed system is continuously withdrawn from the system.

3. A method as claimed in claim 2, characterised in that 5 - 10% of the fluid in the closed system is continuously withdrawn from the system.

4. A method as claimed in claim 1, characterised in that a proportion of the fluid in the system is withdrawn from the system at predetermined time intervals.

5. A method as claimed in any preceding claim, characterised in that, after the make-up liquor has been added to the filtrate and before the filtrate is sterilised, the filtrate is re-filtered.

6. A method as claimed in any preceding claim, characterised in that $CO_2$ is added to the sterilised filtrate prior to the filtrate being returned to the closed system.

7. A method as claimed in any preceding claim, characterised in that the biomass culture in the closed system is operated photosynthetically.

8. A method as claimed in claim 7, characterised in that the synthesis fluid in the closed system is caused to circulate continuously through a substantially transparent tube, the exterior of the tube being exposed to light energy.

9. Apparatus for use with a closed system (1) in which biomass culture takes place under sterile conditions, comprising means (3) for withdrawing a quantity of the synthesis fluid from the system, means (4) for filtering solid biomass product from the fluid, means (5, 6) for adding nutrient make-up liquor to the filtrate, means (7) for sterilising the filtrate and means (12, 13) for returning the fluid to the closed system.

Fig.I.

FIG.2.